# EUROPEAN PATENT APPLICATION

(11) **EP 1 645 306 A1**
(43) Date of publication of application: **12.04.2006**
(21) Application number: 03741312.7
(22) Date of filing: 10.07.2003
(51) Int. Cl.: A61N 1/365

(54) **PACEMAKER SYSTEM FOR TREATING SLEEP APNEA SYNDROME**

(71) Applicant: JMS CO.,LTD., Naka-ku, Hiroshima-shi, Hiroshima 730-8652 (JP)
(72) Inventor: SUGA, Chikashi, Sakado-shi, Saitama 350-0225 (JP); YAMASHITA, Tetsui, c/o JMS CO., LTD., Hiroshima-shi, Hiroshima 730-8652 (JP); TAKANO, Yutaka, c/o JMS CO., LTD., Hiroshima-shi, Hiroshima 730-8652 (JP)
(74) Representative: Flaccus, Rolf-Dieter
(86) International application number: PCT/JP2003/008762
(87) International publication number: WO 2005/004986

(57) **Abstract**

A pacemaker system comprising apnea detecting means for measuring parameters about respiratory including at least two parameters of the number of apneas per unit time and the period of time during which an apnea continues and detecting an apnea state of a sleeping patient on the basis of the measured parameters, **characterized in that** when the measured values of the detection parameters measured by the apnea detecting means increase over reference values at which the pacing rate is changed to a preset and stored SAS treating pacing rate, the pacing rate is changed and the pacing mode is changed to an SAS treating pacing mode. The apnea syndrome of the sleeping patient is detected and treated coping with the bradycardia and various problems accompanying the apnea syndrome. The pacing rate and the pacing rate time can be set suitably for the patient without imposing too heavy a burden on the patient depending on the condition of the patient, and consequently the syndrome of the SAS can be treated efficiently and reliably.

## Description

### Technical Field

The present invention relates to a pacemaker system for detecting apnea symptoms of a patient during sleep, and remedying them coping with bradycardia and various problems accompanying the apnea symptoms.

### Background Art

Sleep apnea syndrome (hereinafter also referred to as SAS) is defined as a symptom in which no less than 30 apneas occur in one night, or apneas lasting for not less than 10 seconds occur at a frequency of not less than 5 times per hour. Such symptoms are classified in terms of causes into three major types : an "obstructive type", which is originated in the nose and throat, a "central type", which is attributed to potential organic or functional disorders in the cerebrum or brain stem, and a "mixed type", which is a combination of the two.

It is known that a high proportion of patients having SAS are likely to suffer bradycardia in combination (sinus bradycardia/atrioventicular block). Such patients often utilize a pacemaker, of which conventional ones are based on an algorithm to perform pacing at a fixed pacing rate or a sensor rate regardless of the presence or absence of the event of breathing/non-breathing. However, in the case of a SAS patient, pacing at such a fixed pacing rate or sensor rate would promote the bradycardia and prolong the circulation time from the lung to the brain due to the decrease in heart rate, eventually causing a decrease in the brain blood flow. As the result, it is said that further prolongation of apnea duration and reduction in oxygen saturation would occur; thus there has been the need for improvements in this respect. Furthermore, a conventional pacemaker has a function of changing the pacing rate for the purpose of recreating the diurnal fluctuation in the patient's heart rate, which may adversely affect the patient having SAS. That is, a conventional pacemaker has a function of setting a pacing rate lower than a normal basic rate during rest (sleep) to reduce the burden on the patient's physiological function during rest, and this function is commonly referred to as a rest rate, a night rate drop, a sleep rate, etc. However, in the case of a patient having SAS, when a state of reduced pacing rate is produced due to the above-described rest rate function or the like, the heart rate, which would be lowered while an apnea occurs, would further decline causing various problems such as the promotion of bradycardia, the reduction in brain bloodflow, etc. Therefore, a pacemaker with a rest rate function was rarely applied to SAS patients and, in case it is applied, a greatest care was required. To cope with suchproblems, studies for adapting a pacemaker to SAS patients have been conducted as described in U.S. Patent No.6126611 and WO01/41868A1, and a pacemaker has been proposed whereby when an apnea during sleep is detected with a sensor, the pacing rate is increased thereby solving the bodily problems accompanying bradycardia and decreased heart beat. However, although the pacemakers disclosed in the above mentioned documents have sensor means as apnea detection means and the reference value of the pacing rate to be adopted upon detecting an apnea is predetermined, the setting of the reference value of the pacing rate was unchangeable and was not adaptable depending on the symptoms of the patient. It is also difficult to cope with a type of sleep apnea, in which thorax activity is not completely diminished such as an obstructive sleep apnea.

### Disclosure of the Invention

The pacemaker system, especially of an embedded-type, according to the present invention is characterized in that the system contains apnea detection means which can measure respiratory related parameters (hereinafter also referred to as detection parameters) including at least two parameters: frequency of apneas per unit time and an apnea duration time, to detect an apnea state of the patient during sleep based on the measurement results, and that the system has a function in which the detection parameter values to provide reference values for whether or not the pacing rate is changed to a SAS treating pacing rate are preset and stored for each patient, and when the measured values of said detection parameters through the apnea detection means become not less than the preset and stored detection parameter values, the pacing rate is changed for shifting into a SAS treating pacing rate. Such function includes a function (also referred to as a SAS feature) in which at the time when the reference values for shifting to the SAS treating pacing mode are confirmed, then pacing rate can be automatically changed to a SAS treating pacing rate set for each individual patient thereby continuously shifting into the SAS treating pacing mode.

In the pacemaker system of the present invention, the above-described detection parameter values to provide reference values for whether or not the pacing rate is shifted to a SAS treating pacing rate are preferably such that only when an apnea state during sleep will adversely affect the patient, the pacing rate is increased to shifting into a SAS treating pacing rate to remedy the patient's symptoms and, otherwise, when the apnea state is mild, the shifting to the SAS treating pacing rate is not carried out so as not to disturb the resting state of the patient.

Accordingly, in the inventive pacemaker system, although the apnea detection means continuously monitors whether a SAS event is occurring or not, the above-described reference values of the apnea detection parameters for shifting into the SAS treating pacing rate in the pacemaker system of the invention are preferably settable taking into consideration individual symptoms depending on the apnea state of the SAS patient instead of those uniformly functioning when a SAS event occurs because depending on each SAS patient, there are cases in which a SAS event will not impair the living or health of the patient unless it becomes a seriously high level, and conversely there are cases in which a relatively mild SAS event will harm the patient.

In the pacemaker system of the present invention, as the judgment indicator to determine whether or not sleep apnea occurred will adversely affect the patient, at least two parameters: frequency of apneas per unit time and an apnea duration time, of the patient are adopted, and in setting these reference values of the apnea detection parameters, a physician takes into consideration various conditions including the states and factors of the patient such as subjective symptoms and the sleep quality of the patient, or effects on the complications, for example, hypertension, cardiac failure, coronary disorder, bradycardiac arhythmia, tachycardiac arhythmia, etc. to preset and store them in the pacemaker system of the present invention. Accordingly, since the pacemaker system of the present invention can perform SAS treating pacing rate as described above, it can remedy the SAS symptoms of a SAS patient and, in addition to that, it will not disturb the resting state of the patient since the pacing rate will not be aimlessly changed in the stage of a mild SAS, thus making it possible to effectively perform SAS treatment without imposing an unnecessary burden to the patient.

Another characteristics of the inventive pacemaker system is that its pacing rate can be set or changed as described below. Such setting or change of the pacing rate can be performed based on new information obtained, for example, through a telemetry function (communication with the pacemaker).
(1) The SAS treating pacing rate to be adopted upon occurrence of an apnea is, as described above, preset so as to be adaptable to each patient and stored in the pacemaker of the present invention by a physician or the like; however, this preset SAS treating pacing rate can be changed after it is stored, based on newly obtained information as described above. Thus, the inventive pacemaker system is able to remedy patient's symptoms more efficiently due to the fact that the SAS treating pacing rate pre-stored in the system is changeable based on newly obtained appropriate information as described above.
(2) The system has a function of properly changing the set rate of the SAS treating pacing rate as desired even after having shifted to the SAS treating pacing. Such configuration makes it possible to change the set rate of the preset SAS treating pacing rate so as to be better suited for patient's symptom at the time when it is found that the adopted SAS treating pacing rate is not adapted for the patient, and consequently to remedy the patient's symptom quickly.
(3) The system has a function of automatically returning the SAS treating pacing rate to a pacing rate typically used for patients (basic pacing rate) if, after having shifted to the SAS treating pacing, measured values of the detection parameters decline below the preset reference values of the detection parameters for shifting into the SAS treating pacing rate as the consequence of performing the SAS treating pacing. Also, when a rest rate during sleep is set, the function of automatically returning to the above-described pacing rate (basic pacing rate) may automatically decrease the SAS treating pacing rate to the rest rate. Further, shifting from a basic pacing rate or a rest rate to the SAS treating pacing rate, or shifting from the SAS treating pacing rate to the basic pacing rate or the rest rate is preferably performed in a gradual manner in either case in order to avoid a rapid change of the heart rate of the patient.

As the apnea detection means for measuring the above-described detection parameters for the inventive pacemaker system, a MV sensor for measuring minute ventilations (hereinafter, also referred to as MV) is suitable since temporal fluctuation result in the measured minute ventilations makes it possible to distinguish a normal sleep state from an apnea state even for an obstructive apnea case in which the both states are difficult to be distinguished. Furthermore, the inventive pacemaker system can readily distinguish an obstructive sleep apnea from a central sleep apnea by being provided with a QT time detection type sensor and/or a sensor capable of identifying heart rate fluctuation patterns in addition to the above-described apnea detection means for measuring the detection parameters.

The inventive pace maker system preferably contains, in addition to the above-described apnea detection means for measuring the detection parameters, an acceleration (ACC) sensor or a body motion (Activity) sensor as the sleep detection means for detecting a sleep state of the patient by measuring the body motion of the patient. By using the above-described acceleration (ACC) sensor or body motion (Activity) sensor in connection with the above-described apnea detection means, it is possible to improve the reliability of the detection of an apnea state of the patient compared with the case in which only the above-described apnea detection means is used.

Another characteristics of the pacemaker of the present invention is that it has storage means (a memory) for storing information as shown below.
(1) Detection parameter values measured by the above-described apnea detection means, sensing results or measured values by the acceleration (ACC) sensor or the body motion (Activity) sensor, and the fluctuation history of each of the foregoing data.
   For example, by storing the fluctuation results of MV values captured by the sensing with the MV sensor, which is the above-described apnea detection means, in the above-described storage means (a memory) and utilizing an algorithm capable of identifying whether the patient is in a normal sleep state or in an apnea state based on the fluctuation result of, for example, the respiratory cycle or intensity of the patient, it is possible to reliably distinguish n a normal sleep state from an apnea state even for obstructive apnea cases in which it is difficult to discriminately detect a normal sleep state or an apnea state solely through the apnea detection parameters of the apnea detection means. Moreover, when an apnea state is identified in the above-described manner, it is possible to determine whether the apnea state is an obstructive type or a central type from the temporal fluctuation result of the measured minute ventilations. Therefore, based on the above mentioned determination, it is possible to take a more appropriate countermeasure against each type of apnea, and thus the symptoms of the patient are expected to be further remedied.
(2) History relating to the above-described SAS treating pacing rate
   The history relating to the SAS treating pacing rate includes parameters such as time zones in which SAS took place and in which SAS ended, the number of the events, adopted pacing rates, and the like. By storing the above-described parameters in storage means, it becomes possible to confirm when the operation of SAS feature was needed or not needed, and to confirm the treatment effect by the application of SAS treatment pacing rate at that time, and further to effectively modify and reset the reference values of the above-described detection parameters.

The pacing rate to be adopted in the inventive pacemaker system will be described in detail below.
(1) The pacing rate for the instant when an apnea occurs includes a basic pacing rate, which is the fundamental set rate, and a rest rate, which is not necessarily to be set, but can specifically be set during sleep and is lower than or equal to the pacing rate during arousal.
(2) If the pacing rate at the time when a SAS event is detected is the basic pacing rate, the SAS treating pacing rate will be, of course, higher than the basic pacing rate.
(3) If the pacing rate at the time when a SAS event is detected is the above-described rest rate, the SAS treating pacing rate will be higher than the above-described rest rate; however, the pacing rate in this case may be higher than the basic pacing rate, or may be higher than the above-described rest rate but lower than the basic pacing rate.
(4) In the case in which no pacing is being performed during sleep, a pacing rate higher than the heart rate of the patient at the time when a SAS event occurs is adopted as a SAS treating high rate.

Since in most cases the pacemaker system has been considered to be adapted for bradycardiac arrythmias, the principal target of the present invention is assumed to be cases in which the patient has bradycariac arrythmias in need of pacemaker treatment and also suffers SAS in combination. Accordingly, in this specification, the present invention has been described only in the case of patients who suffer SAS and bradycardiac arrythmias in combination. However, the present invention is not necessarily intended for the cases in which SAS and bradycardiac arrythmias take place, but can also be applied to the cases of SAS alone without complication by bradycardia.

### Brief Description of the Drawings

Fig. 1 is a flowchart to illustrate the mechanisms of SAS detection and SAS pacing according to the pacemaker system of the present invention.
Fig. 2 is an illustration in time of the SAS therapy according to the pacemaker system of the present invention.

### Best Mode for Carrying out the Invention

The pacemaker system of this embodiment, which is configured as shown below, is adapted for models with various modes such as SSI(R), VDD(R), DDD(R), etc.

An electric circuit for measuring thorax impedance through a lead embedded in the body is contained in the main body of the pacemaker. The electric circuit for measuring thorax impedance, which is part of a MV sensor, provides apnea detection means. A storage circuit for storing a MV sensor histogram is contained in the main body of the pacemaker. Although the pacemaker of this embodiment is configured such that when the rest rate is turned on, the SAS feature is automatically turned on, the SAS feature can also be set to be ON or OFF independently regardless of whether the rest rate is set or not. Further, in the pacemaker system of this embodiment, as the reference values for shifting into a SAS pacing mode, reference values, for example, not less than 5 SAs per hour and not shorter than 10 seconds for one SA are predetermined; however, these values can be changed by a physician. When a SA having values not lower than the set reference values takes place, the pacemaker system of this embodiment changes the pacing rate by way of the SAS feature, automatically shifting into the SAS pacing mode. The above-described SAS feature has a function of turning ON or OFF of automatically setting the time period during which SAS pacing rate is performed when automatically shifting into the SAS pacing mode. When automatically shifting into the SAS pacing mode by way of the ON set of the above-described function of automatically setting the time period for performing the above-described SAS pacing rate, the pacing rate and/or pacing time period adopted in the SAS pacing mode is predetermined for each patient and stored in the pacemaker system. When automatically shifting into the SAS pacing mode in the above-described ON set, the pacing is performed at the SAS pacing rate for the set time period, and when automatically shifting into the SAS pacing mode in the above-described OFF set, the SAS pacing is performed until the SAS state is resolved and the pacing in the SAS pacing mode is discontinued at the time when SAS state is resolved due to the SAS pacing thus returning to a normal basic pacing rate or a rest rate set for each patient during sleep. However, the system may also have a function of continuously performing the pacing in the SAS pacing mode for the time period, for example, set by the above-described ON set even if the SAS state has been resolved; by continuously performing such pacing, it is made possible to achieve effects such as the prevention of serious sleep apneas and the prevention of arousal of the patient due to frequent changes of the heart rate. Furthermore, it would also be possible to provide a function of automatic SAS monitor function being turned on, which allows confirmation of frequency of SA per hour and duration time of an individual SA.

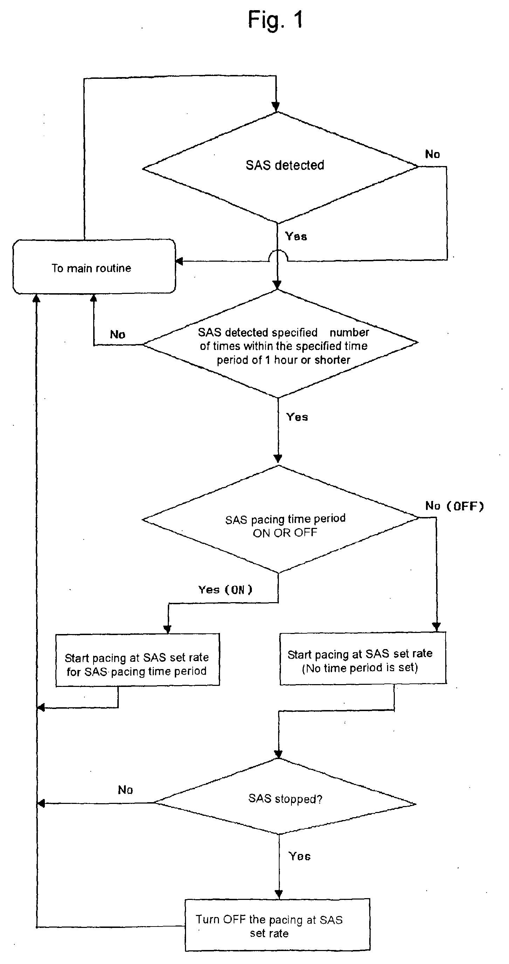

As shown in Fig. 1, the absence or presence of a sleep apnea (SA) of the patient is confirmed by the SAS detection means . If no SA is detected by the above-described detection means (No), the process goes into a main routine keeping the current rate. On the other hand, if the SAS detection means detects a SA (Yes), it is confirmed whether SAs of a set apnea time period are detected set number of times within a set time period by the above-described detection means (SAS detected specified number of times within a specified time period of one hour or shorter?) . If the confirmation result shows that SAs of the set apnea time period are not detected set number of times (No), it will be determined that there is no need of shifting into a SAS pacing mode and thus the process goes into a main routine at the same pacing rate as the current SAS pacing rate. If the detection result by the detection means exceeds the reference values which are preset and stored in the pace maker system and at which the pacing mode is shifted to the SAS pacing mode, the pacing mode is automatically shifted into a SAS pacing mode by the SAS feature (SAS pacing period ON OR OFF) . This SAS feature allows for the selection of modes for turning ON and OFF the function of automatically setting the SAS pacing time period, and if the ON mode is selected (Yes), the SAS pacing is successively performed automatically at a set pacing rate for a set time period (pacing at the SAS set rate for the SAS pacing time period), and if the OFF mode is selected (No), SAS pacing is performed by changing the pacing rate to a preset and stored SAS pacing rate by the above-described SAS feature [(start pacing at the SAS set rate (no setting of time period)], and although SAS pacing rate is maintained for the time period during which the above-described detection means is detecting SAs (No), at the time when the detection means becomes not to detect a SA (Yes), that is when the detection means confirms that the patient's SAS state has been resolved, the pacing at the SAS set rate is stopped (turn OFF the pacing at the SAS set rate), going into a main routine.

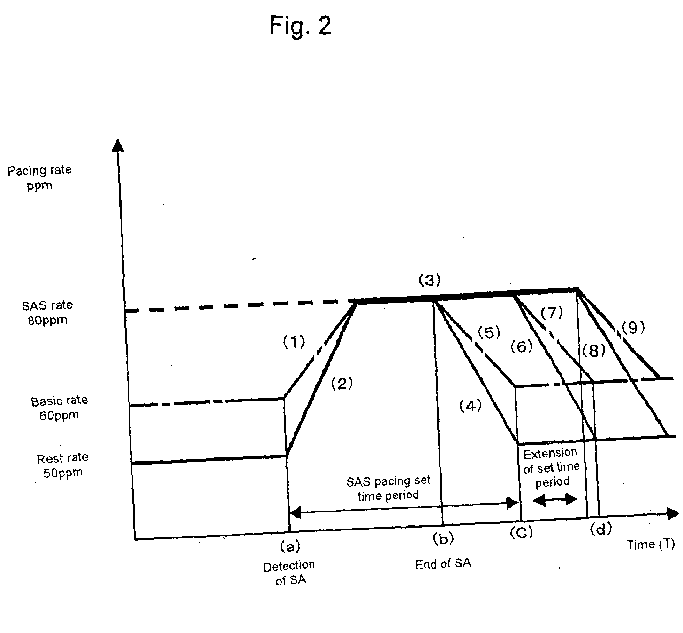

In Fig. 2, the chain line shows a pacing history in the SAS therapy at a basic rate of 60 pulses/minute (60 ppm) as the basic rate during sleep; and the solid line shows a pacing history in the SAS therapy at a rest rate of 50 pulses/minute.

Since a SAS state to be treated was detected at time (a), the pacing rate was successively increased (route (1) or route (2.)), and SAS therapy was conducted at a SAS pacing rate of 80 pulses/minute. As the result, at time (b), the resolution of SA state was detected, and the pacing rate was successively returned to the basic rate or the rest rate adopting either of the two methods shown below.
(1) At the time when the resolution of a sleep apnea (SA) state is detected, that is the time when measured results of the detection parameters of the apnea detection means become not higher than the reference values for shifting into the SAS treating pacing rate [time (b) ], the pacing rate is successively reduced, immediately following route (4) or route (5), returning to a basic rate of 60 pulses/minute or a rest rate of 50 pulses/minute at time (c).
(2) At the time when the resolution of a sleep apnea (SA) state is detected, that is the time when the measured results of the detection parameters by the apnea detection means become not higher than the reference values for shifting into the SAS treating pacing rate [time (b)], instead of immediately reducing the pacing rate to the basic rate or the rest rate, the SAS treating is continued at the SAS pacing rate for a fixed time period, for example, the automatically set SAS pacing time period, and from time (c) when the set SAS pacing time period is elapsed, the pacing rate is successively reduced following route (6) or route (7) returning to the basic rate of 60 pulses/minute or the rest rate of 50 pulses/minute. The foregoing SAS treating method, compared to the treating method (1), will achieve the effects such as the prevention of serious sleep apnea and the prevention of arousal reaction of the patient due to frequent changes in heart rate, by continuing the SAS pacing within the set SAS pacing time period even if a sleep apnea (SA) state is resolved.

Furthermore, if a SAS pacing time period has been automatically set, and when a SA state is being detected at a time immediately before the expiration of the set period, it is possible to continue the SAS pacing rate even after the expiration of the set period by extending the set period instead of discontinuing the SAS pacing rate and, at the time when an end of SA is detected, immediately from that time the SAS pacing rate is gradually reduced following the route (7) or route (8) as described above. In this case, instead of performing the operation of successively reducing the pacing rate immediately after the time when an end of SA is detected, it is also possible to perform the operation after an elapse of the fixed time period as in the above-described method (2) (although this case is not shown in Fig. 2).

### Industrial Applicability

As so far described, the pacemaker system according to the present invention contains apnea detection means which can measure respiratory related parameters including at least two parameters: frequency of apneas per unit time and a duration time period for which the apnea has continued, to detect an apnea state of the patient during sleep based on the measurement results; and is characterized in that if the measured values of the respective detection parameters through the apnea detection means become not lower than a preset and stored reference values for shifting into the SAS treating pacing mode, the pacing rate can be changed shifting into a SAS treating pacing mode; the system is suitable for detecting apnea symptoms of the patient during sleep and remedying them, coping with bradycardia and various problems accompanying the apnea symptoms; and the system can securely and effectively remedy the symptoms due to SAS since it is possible to set a pacing rate and a pacing rate time period adapted for individual patient without giving an excessive burden depending on patient's conditions.

## Claims

1. A pacemaker system **characterized in that** said system contains apnea detection means that can measure respiratory related parameters (detection parameters) including at least two parameters: frequency of apneas per unit time and an apnea duration time, to detect an apnea state of a patient during sleep based on saidmeasurement results, and that if the measured values of said detection parameters through said apnea detection means increase not lower than preset and stored reference values for shifting to SAS pacing rate, a pacing rate can be changed for shifting into a SAS treating mode.

2. The pacemaker system according to claim 1, **characterized in that** said apnea detection means is a MV sensor.

3. The pacemaker system according to claim 1 or 2, **characterized in that** said system has.a function (SAS feature) in which at a time when the reference values for shifting into the SAS treating pacing mode are confirmed, a current pacing rate can be automatically changed to the SAS treating pacing rate set for each individual patient thereby continuously shifting into the SAS treating pacing mode.

4. The pacemaker system according to claim 3, **characterized in that** said SAS feature has a function of automatically setting a SAS pacing time period, and said function can select ON or OFF mode.

5. The pacemaker system according to claim 4, **characterized in that** if the ON mode of said function of automatically setting the SAS pacing time period for said SAS feature is selected, SAS pacing is performed by automatically changing the pacing rate to the SAS pacing rate preset for each patient for a time period automatically set by the SAS feature; and if the OFF mode of said function of automatically setting the SAS pacing time period for said SAS feature is selected, SAS pacing is continually performed by changing the pacing rate to the SAS pacing rate preset for each patient for a time period while SAs are being detected by said apnea detection means and at a time when measured results of the detectionparameters of said apnea detection means become not higher than the detection parameter values as the reference values for shifting to said SAS treating pacing rate, the pacing at said set SAS pacing rate is stopped and returned to a normal basic pacing rate or a rest rate.

6. The pacemaker system according to claim 5, **characterized in that** immediately after the measured results of the detection parameters of said apnea detection means become not higher than the detection parameter values as the reference values for shifting to said SAS treating pacing rate, the pacing rate is successively reduced back to a basic rate or a rest rate.

7. The pacemaker system according to claim 5, **characterized in that** at the time when the measured results of the detection parameters of said apnea detection means become not higher than the detection parameter values as the reference values for shifting to said SAS treating pacing rate, the SAS treating is, in stead of immediately being reduced to a basic rate, continued at the SAS pacing rate for a fixed time period and after the expiration of said fixed time period, the pacing rate is gradually reduced back to the basic rate or the rest rate.

8. The pacemaker system according to claim 5, **characterized in that** said fixed time period is a SAS pacing time period automatically set upon shifting to the SAS pacing.

9. The pacemaker system according to claim 1, 2, 3, 4, 5, 6, 7, or 8, **characterized in that** said detection parameter values as the reference values for shifting to said SAS treating pacing rate are set based on detection parameter values indicating an apnea state adversely affecting the patient.

10. The pacemaker system according to claim 9, **characterized in that** said apnea state adversely affecting the patient is an apnea state affecting complications in the patient.

11. The pacemaker system according to claim 1, 2, 3, 4, 5, 6, 7, 8, 9, or 10, **characterized in that** said system contains a QT sensor and/or a sensor for identifying heart rate fluctuation patterns.

12. The pacemaker system according to claim 1, 2, 3, 4, 5, 6, 7, 8, 9, 10, or 11, **characterized in that** said system contains sleep detection means for detecting the patient asleep by measuring the body motion of the patient.

13. The pacemaker system according to claim 12, **characterized in that** said sleep detectionmeans is an acceleration (ACC) sensor of a body motion (Activity) sensor.

14. The pacemaker system according to claim 1, 2, 3, 4, 5, 6, 7, 8, 9, 10, 11, 12, or 13, **characterized in that** said system contains storage means (a memory) which stores the fluctuation history of the detection or sensing results of at least one of said apnea detection means for measuring said detection parameters, said acceleration (ACC) sensor or body motion (Activity) sensor, said QT sensor, said sensor for identifying heart rate fluctuation patterns, and said SAS feature.

15. The pacemaker system according to claim 14, **characterized in that** said system can securely determine whether the patient is in a normal sleep state or in an apnea state by utilizing the fluctuation history of a MV sensor as an apnea detection means.

16. The pacemaker system according to claim 14 or 15, **characterized in that** said system determines whether the patient is in a normal sleep state or in an apnea state based on a period and intensity of the patient's respiration in fluctuation of measured values of the detection parameters of said apnea detection means stored in said storage means (memory).

17. The pacemaker system according to claim 14, 15, or 16, **characterized in that** said system can distinguish between an obstructive sleep apnea (OSA) and a central sleep apnea (CSA) based on the fluctuation history of the detection or sensing results of the MV sensor as a sleep apnea detection means, stored in said storage means (memory) and the fluctuation pattern of said QT sensor and/or the fluctuation history of the detection or sensing results of said sensor for identifying heart rate fluctuation patterns.
